(19) Europäisches Patentamt
European Patent Office
Office européen des brevets

(11) Publication number: **0 223 124 B1**

(12) # EUROPEAN PATENT SPECIFICATION

(45) Date of publication of patent specification: **12.06.91**

(51) Int. Cl.5: **C07D 211/42**, C07D 211/46, C07D 207/12, A61K 31/395

(21) Application number: **86114995.3**

(22) Date of filing: **29.10.86**

The file contains technical information submitted after the application was filed and not included in this specification

(54) **Substituted aminosulfonyl 6-nitrobenzoic-esters or amides, processes for their preparation and pharmaceutical compositions containing them.**

(30) Priority: **06.11.85 US 795567**

(43) Date of publication of application: **27.05.87 Bulletin 87/22**

(45) Publication of the grant of the patent: **12.06.91 Bulletin 91/24**

(84) Designated Contracting States: **CH DE FR GB IT LI NL**

(56) References cited:
**EP-A- 0 054 898**
**EP-A- 0 088 849**
**FR-A- 2 343 736**

**JOURNAL OF MEDICINAL CHEMISTRY, vol. 14, no. 4, April 1971, pages 350-354; J.R. PIPER et al.: "Terminal dicarboximido analogs of S-2-(omega-aminoalkylamino)ethyl dihydrogen phosphorothioates and related compounds as potential antiradiation agents. 2. Succinimides, glutarimides, and cis-1,2-cyclohexanedicarboximides"**

**JOURNAL OF MEDICINAL CHEMISTRY, vol. 12, no. 2, March 1969, pages 244-253; J.R. PIPER et al.: "Terminally substituted S-2-(omega-aminoalkylamino)ethyl dihydrogen phosphorothioates and related compounds as potential antiradiation agents"**

(73) Proprietor: **MERCK & CO. INC.**
**126, East Lincoln Avenue P.O. Box 2000**
**Rahway New Jersey 07065-0900(US)**

(72) Inventor: **Engelhardt, Edward L.**
**904 Plymouth Road**
**Gwynedd Valley, PA 19437(US)**
Inventor: **Saari, Walfred S.**
**1740 Wagon Wheel Lane**
**Lansdale, PA 19446(US)**

(74) Representative: **Blum, Rudolf Emil Ernst et al**
**c/o E. Blum & Co Patentanwälte Vorderberg 11**
**CH-8044 Zürich(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**Description**

BACKGROUND OF THE INVENTION

This invention relates to esters, amides and N-substituted amides of 2-[N-( piperidinoalkyl) and ( pyrrolidinoalkyl)aminosulfonyl]-6-nitrobenzoic acids and salts of said compounds. The corresponding compounds accordingly are esters or amides of the benzoic acid which are substituted in the o-positions to the ester group, respectively acid amide group with a nitro group as well as an amino sulfonyl group in which there is bonded to the nitrogen atom of the amino sulfonly group an alkyl chain comprising 2 or 3 carbon atoms which is substituted in the ω-position with the nitrogen atom of a piperidino or pyrrolidino ring which heterocycle is optionally substituted with 1 or 2 hydroxy groups. The corresponding esters or amides of the benzoic acid are useful as sensitizers of hypoxic tumor cells to therapeutic radiation.

The invention furthermore concerns pharmaceutical compositions for enhancing the effect of a therapeutic radiation of hypotoxic tumor cells, which compositions contain as pharmaceutically active ingredient the above stated esters or amides of the benzoic acid which are substituted in both ortho-positions of the benzene nucleus. The corresponding pharmaceutical compositions are administered to patients in need of a therapeutic radiation treatment of hypoxic tumor cells.

DESCRIPTION OF THE PRIOR ART

At the present time, certain other unrelated compounds are in experimental clinical use as radiation sensitizers. Said non-related compounds are for example 2-nitro-imidazole derivatives, like the compounds named metronidazol and misonidazole.

Said compounds however suffer from the drawback, that they also cause neurotoxicity which limits their usefulness.

In the U.S. patent 4 396 622 there are described sulfonamides which have a completely different pharmaceutical activity, i.e. they have an anti-arrhymthmic activity. The amino moiety of said sulfonamides which are claimed in said U.S. patent is derived from ethyl amine or n-propyl amine which is substituted in its ω-position with a cyclic amino radical which is selected from the group consisting of pyrrolidino, 2,4-dimethyl-pyrrolidino, 2,5-dimethyl-pyrrolidino, 2,4-dimethyl-piperidino and 4-methylpiperazino.

According to the abstract of disclosure and the table of said U.S. patent there are disclosed corresponding sulfonamide compounds which are free of the trifluoromethyl substituent and which compounds in addition to the sulfonamide group can either have a nitro substituent or a carboxamide substituent in the benzene nucleus (see the definition of the radical $R^1$ in column 1, lines 42 - 46 and the compound no. 25 in the table I).

DETAILED DESCRIPTION OF THE INVENTION

It was the aim of the present invention to provide compounds which are useful as radiation sensitizers and which do not have the undesired side effects of until now used radiation sensitizers. It was surprisingly found out that new esters or acid amides of the benzoic acid, which have a nitro substituent in the 6-position and an amino sulfonyl substituent in the 2-position to said ester group or acid amide group have the desired properties.

One object of the present invention are accordingly substituted amino sulfonyl 6-nitrobenzoic esters or amides of formula I

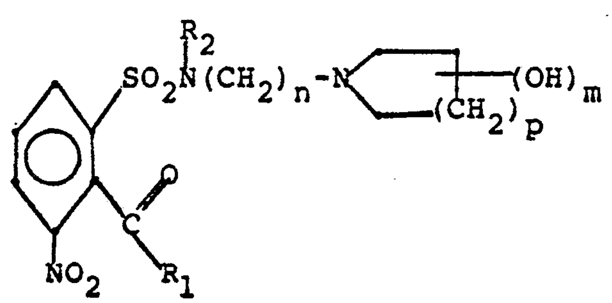

wherein

$R_1$ is hydroxy-alkoxy having 1 - 4 carbon atoms, alkoxy having 1 - 4 carbon atoms, allyloxy or a group having the formula

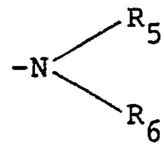

wherein
$R_5$ and $R_6$ are independently from each other hydrogen, alkyl having 1 - 4 carbon atoms or hydroxy-alkyl having 1 - 4 carbon atoms or one of said radicals is allyl and the other one is hydrogen;
$R_2$ is hydrogen, alkyl having 1 - 4 carbon atoms, hydroxy alkyl having 1 - 4 carbon atoms or allyl;
n is 2 or 3;
m is 0, 1 or 2;
p is 1 or 2
or salts of the compounds of formula I.

In preferred inventive compounds of formula I m is 1.

A further object of the present invention is a process for the preparation of the inventive compounds of formula I and salts thereof, which process is characterized in that either a compound of formula III,

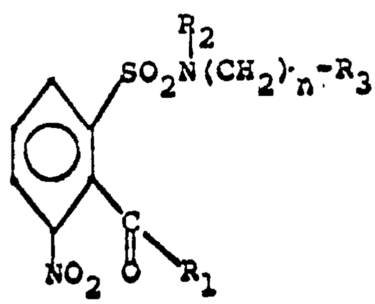

III

wherein $R_3$ is a displaceable group and $R_1$, $R_2$ and n are as defined in formula I, is reacted in an aprotic solvent with an amine of formula IV

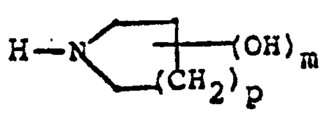

IV

wherein p and m have the same meaning as in formula I yielding the compounds of formula I, which are isolated in the free form or as salts thereof, or that a 2-halosulfonyl compound of formula V

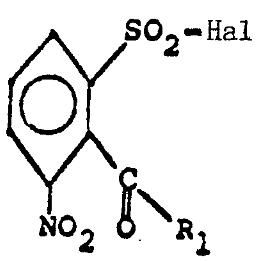

V

wherein
Hal is chlorine and
$R_1$ is as defined in formula I,
is reacted with an amine of formula VI

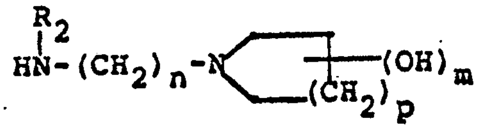 VI

wherein $R_2$, n, p and m are as defined informula I yielding the compounds of formula I which are isolated in the free form or as salts thereof, and that optionally a prepared compound of formula I in which
$R_1$ is hydroxy-alkoxy having 1 - 4 carbon atoms or alkoxy having 1 - 4 carbon atoms is further reacted with ammonia or an amine of formula VII

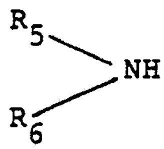 VII

wherein
$R_5$ and $R_6$ are independently from each other hydrogen, alkyl having 1 - 4 carbon atoms or hydroxy-alkyl having 1 - 4 carbon atoms or one of said radicals $R_5$ and $R_6$ is hydrogen and the other one is allyl, yielding compounds of formula I in which
$R_1$ is a group having the formula

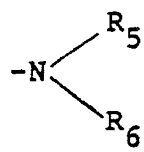

and that said benzoic acid amides are isolated in the free form or as salts thereof.

According to said process in the used starting material of formula III the displaceable group $R_3$ is preferably halogen, alkylsulfonyl or arylsulfonyl
Furthermore, if the synthesis is performed so that an amine of formula IV or an amine of formula VI takes part then said reaction is preferably performed in the presence of a base.

The starting material having the formula III which has the displaceable group $R_3$ can be prepared from the corresponding alcohols, which have a group -OH instead of the displaceable group $R_3$ by established methods. The corresponding alcohols which are applicable for the preparation of the starting materials of formula III are already described in the U.S. patent no. 4 654 369.

The preparation of the starting material of formula III as well as its reaction with the amine of formula IV will be further illustrated with the following reaction scheme:

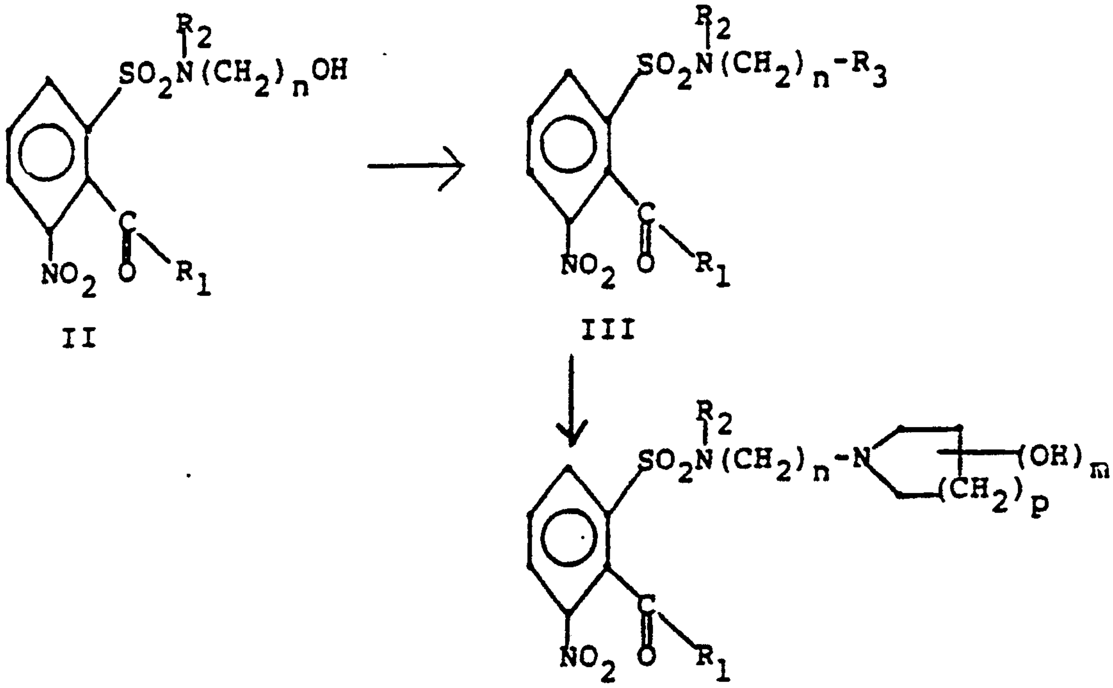

II

III

I

$R_1$, $R_2$, n, m and p are as defined hereinabove and $R_3$ is a displaceable group such as halogen, alkylsulfonyloxy or arylsulfonyloxy.

The reaction is carried out in a suitable aprotic solvent such as dimethylformamide, acetonitrile or the like. The reaction temperature may vary from 50 °C to the boiling point of the solvent for a period of 1 to 10 days. It is preferred to carry out the reaction in the presence of a base in sufficient amount to neutralize the acid formed in the course of the reaction. The base may be a tertiary amine such as a trialkylamine or pyridine. Alternatively, at least twice the molar amount of reactant amine theoretically required may be used. In this event, the reactant amine is utilized both to form the desired product and to neutralize the acid formed in the amination reaction.

If the inventive compounds of formula I are prepared by reacting the corresponding 6-nitro substituted ester or amide of the benzoic acid of formula V which has the chlorosulfonyl substituent in the position 2 with the amine of formula VI, then said compound of formula V is preferably reacted with at least an equimolar amount of the amine of formula VI

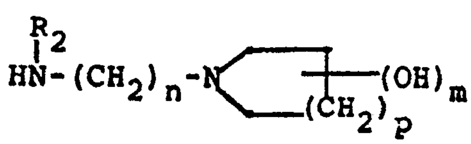

VI

wherein $R_2$, n, m, and p are as described hereinabove.

It is preferred to carry out the reaction in the presence of a base in sufficient amount to neutralize the hydrogen chloride formed in the course of the reaction. The base utilized may be a tertiary amine such as triethylamine or pyridine. On the other hand the same results may be produced by adding at least twice the molar amount of reactant amine theoretically required. In this event, the reactant amine is utilized both to form the sulfonamide and to neutralize the hydrogen chloride formed in the amination reaction.

The temperature at which the reaction is carried out is not critical and may vary from 0 °-100 °C or at the reflux temperature of the solvent, if under 100 °C. The reaction temperature is preferably maintained at about 0-25 °C for a period of 1 - 24 hours. The amination reaction of the chlorosulfonyl compound of formula V with the amine of formula VI can be illustrated with the following reaction scheme:

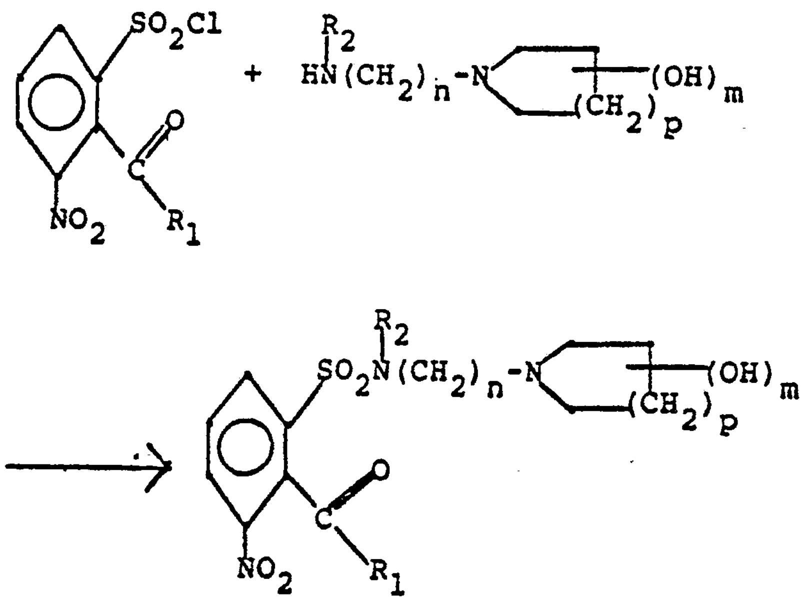

The amination reaction of a compound of formula I wherein $R_1$ is hydroxyalkoxy having 1 - 4 carbon atoms or alkoxy having 1 - 4 carbon atoms with ammonia or an amine of formula VII can be illustrated with the following reaction scheme:

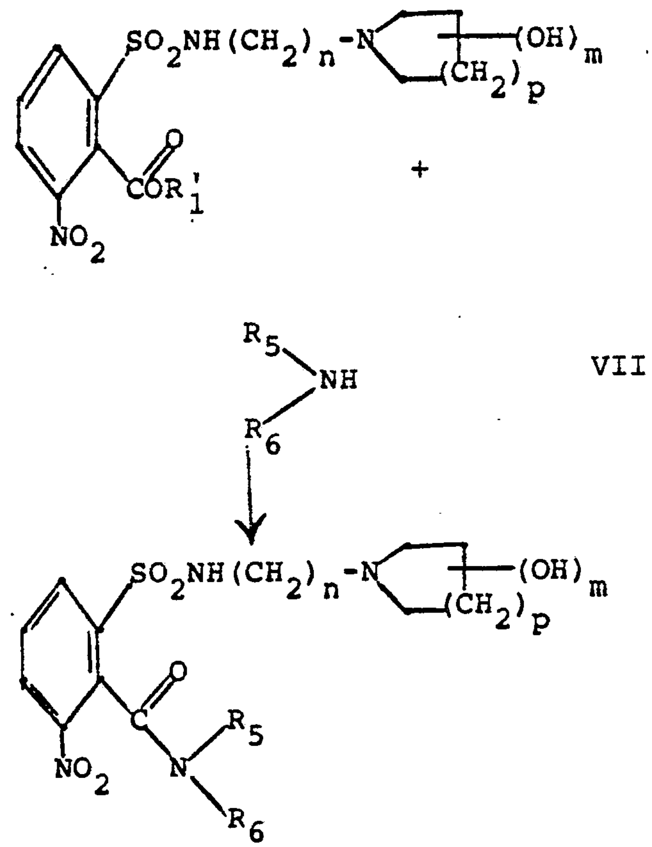

Ia

VII

Ib

In the corresponding ester starting material of formula Ia $R_1'$ is hydroxy-alkoxy having 1 - 4 carbon atoms or alkoxy having 1 - 4 carbon atoms. Said ester is reacted with ammonia or an amine of formula VII in which $R_5$ and $R_6$ are independently from each other hydrogen, alkyl having 1 - 4 carbon atoms, hydroxy-alkyl having 1 - 4 carbon atoms or allyl. Examples for corresponding compounds of formula VII are

accordingly ammonia, alkylamine having 1 - 4 carbon atoms in the alkyl group, dialkylamine having 1 - 4 carbon atoms in each alkyl group, hydroxy alkyl amine having 1 - 4 carbon atoms, di(hydroxy alkyl)-amine having 1 - 4 carbon atoms in each hydroxy alkyl group, (hydroxyalkyl)-alkylamine having 1 - 4 carbon atoms in the alkyl respectively hydroxyalkyl group as well as allyl amine.

The reaction illustrated with the above reaction scheme is carried out in a suitable solvent such as a lower aliphatic alcohol or a polar aprotic solvent such as dimethylformamide, dimethylsulfoxide or others such as tetrahydrofuran, glyme, or diglyme. The reaction temperature is not critical and may vary from 0-100° C, preferably from about 25° - 50° C for a period of 1 to 10 days. When low boiling amines are used, the reaction may be run in a sealed vessel.

A further object of the present invention is a pharmaceutical composition for enhancing the effect of a therapeutic radiation of hypoxic tumor cells, which composition is characterized in that it contains as pharmaceutically active ingredient a compound of formula I or a pharmaceutically acceptable salt of the compound of formula I.

Preferred pharmaceutical compositions contain as active ingredient a corresponding compound of formula I wherein m is 1. Usually the pharmaceutical compositions contain as further component a pharmaceutically acceptable carrier.

In the inventive compounds of formula I the alkyl groups, respectively alkoxy groups having 1 - 4 carbon atoms can be either straight or branched chain.

The following examples are intended to illustrate but not to limit the process of preparation, products, compositions or methods of treatment aspects of the invention. Temperatures are in degrees Celsius unless otherwise indicated throughout the specification.

EXAMPLE 1

N,N-Dimethyl-2-[N-methyl-N-(2-(4-hydroxypiperidino)ethyl)aminosulfonyl]-6-nitrobenzamide hydrogen oxalate

Step A: N,N-Dimethyl-2-[N-(2-methylsulfonyloxyethyl)-N-methylaminosulfonyl]-6-nitrobenzamide

Methanesulfonyl chloride (0.24 ml) was added to a solution of N,N-dimethyl-2-[N-(2-hydroxyethyl)-N-methylaminosulfonyl]-6-nitrobenzamide (500 mg, 1.51 mmol) in dry pyridine (5 ml) and the reaction mixture was stirred at 20-25° for 4 hours. After concentrating under reduced pressure, the residue was partitioned between EtOAc and 1N HCl. The organic extract was washed (saturated NaCl solution), dried ($Na_2SO_4$), filtered and concentrated. The crude product was flash chromatographed over silica gel and eluted with $CHCl_3$ to give pure mesylate (600 mg, 97%) as an oil.

Step B: N,N-Dimethyl-2-[N-methyl-N-(2-(4-hydroxypiperidino)ethyl)aminosulfonyl]-6-nitrobenzamide hydrogen oxalate

A solution of the mesylate (310 mg, 0.76 mmol) and 4-hydroxypiperidine (0.23 g, 2.3 mmol) in THF (20 ml) was stirred at reflux for 2.5 days and then concentrated under reduced pressure. The residue was partitioned between EtOAc and a saturated aqueous NaCl solution. After drying the EtOAc extract over $Na_2SO_4$, filtering and concentrating, the residue was flash chromatographed over silica gel. Product was eluted with 10% MeOH-90% $CHCl_3$ and purified by recrystallization of the hydrogen oxalate salt, m.p. 176.5-178.0° dec, from MeOH-EtOAc-hexane.

EXAMPLE 2

Methyl 2-[N-(2-(4-Hydroxypiperidino)ethyl)aminosulfonyl]-6-nitrobenzoate

Step A: 4-Hydroxy-1-[2-(propionylamino)ethyl]piperidine

A mixture of N-(2-chloroethyl)propanamide (27.1 g, 0.20 mol), 4-hydroxypiperidine (20.2 g, 0.20 mol) and anhydrous sodium carbonate (10.6 g, 0.10 mol) in acetonitrile (120 ml) was stirred at reflux under nitrogen for 20 hours. Additional 4-hydroxypiperidine (20.2 g, 0.20 mol) was added and the mixture stirred at reflux for 3 days. After concentrating under reduced pressure, the residue was dissolved in ethyl acetate and a minimum amount of water. The aqueous layer was saturated with sodium chloride and re-extracted several times with ethyl acetate. The organic extracts were combined, dried ($Na_2SO_4$), filtered and concentrated to give 13.1 g of crude product. Pure product was obtained by flash chromatography over silica gel and elution with 20% methanol-80% chloroform.

Step B: 1-(2-Aminoethyl)-4-hydroxypiperidine dihydrochloride

A solution of 4-hydroxy-1-[2-(propionylamino)ethyl]piperidine (1.7 g, 8.5 mmol) in 10% hydrochloric acid was stirred at reflux for 20 hours and then concentrated under reduced pressure. The residue was triturated with ethyl acetate and the insoluble solid recrystallized from methanol-ethylacetate to give 1.3 g of the dihydrochloride salt, m.p. 186-188° C.

Step C: Methyl 2-[N-(2-(4-hydroxypiperidino)ethyl)amino)sulfonyl]-6-nitrobenzoate

A solution of methyl 2-chlorosulfonyl-6-nitrobenzoate (1.5 g, 5.4 mmol) in tetrahydrofuran (100 ml) was added over 1 hour to a stirred mixture of 1-(2-aminoethyl)-4-hydroxypiperidine dihydrochloride (1.2 g, 5.4 mmol) and triethylamine (2.3 ml, 16.5 mmol) in tetrahydrofuran (75 ml) cooled in an ice bath. After stirring at room temperature for 6 hours, solvent was removed under reduced pressure and the residue partitioned between ethyl acetate and saturated sodium chloride solution. The ethyl acetate extract was dried ($Na_2SO_4$), filtered and concentrated. The residue was flash chromatographed on silica gel and eluted with 5% methanol-95% chloroform to give pure product, 1.0 g, as an oil.

EXAMPLE 3
N,N-Dimethyl 2-[N-(2-(4-hydroxypiperidino)ethyl)aminosulfonyl]-6-nitrobenzamide hydrogen oxalate

A solution of methyl 2-[N-(2-(4-hydroxypiperidino)ethyl)aminosulfonyl]-6-nitrobenzoate (1.0 g, 2.6 mmol) and 40% aqueous dimethylamine solution (3.0 ml) in methanol (50 ml) was stirred at 20-25° for 3 days. After concentrating under reduced pressure, residue was partitioned between ethyl acetate and saturated sodium bicarbonate solution. The ethyl acetate extract was washed with saturated sodium chloride solution, dried ($Na_2SO_4$), filtered and concentrated. The residue was flash chromatographed over silica gel and eluted with 7% methanol-93% chloroform to give 800 mg of pure product as an oil. The base was converted to the hydrogen oxalate salt with oxalic acid and recrystallized from methanol-ethyl acetate-hexane to give analytically pure material, m.p. 80° dec.

Example 4
N,N-Dimethyl 2-[N-methyl-N-(2-(3-hydroxypiperidino)ethylaminosulfonyl]-6-nitrobenzamide Hydrogen Fumarate

A solution of N,N-dimethyl 2-[N-(2-methylsulfonyloxyethyl)-N-methylaminosulfonyl]-6-nitrobenzamide (0.43 g, 1.05 mmol) and 3-hydroxypiperidine (0.31 g, 3.06 mmol) in acetonitrile (40 ml) was stirred at reflux for 1 day. After concentrating under reduced pressure, the residue was partitioned between ethyl acetate and a saturated aqueous solution of sodium chloride. The ethyl acetate extract was dried ($Na_2SO_4$), filtered and concentrated. Flash chromatography of the residue over silica gel and elution with 5% methanol -95% methylene chloride gave 400 mg of pure product as an oil. The hydrogen fumarate salt, m.p. 132-35° dec, was prepared for analysis.

Example 5
N,N-Dimethyl-2-[N-(2-(3-hydroxypyrrolidino)ethyl)-N-methylaminosulfonyl]-6-nitrobenzamide.

A solution of N,N-dimethyl 2-[N-(2-methylsulfonyloxyethyl)-N-methylaminosulfonyl]-6-nitrobenzamide (0.70 g, 1.7 mmol) and 3-pyrrolidinol (0.30 g, 3.4 mmol) in acetonitrile (30 ml) was stirred at reflux for 1 day. After concentrating under reduced pressure, the residue was partitioned between ethyl acetate and a saturated aqueous solution of sodium chloride. The ethyl acetate extract was dried ($Na_2SO_4$), filtered and concentrated to 0.44 g of oily product, homogeneous tlc (5% methanol-95% chloroform, silica gel), $R_f = 0.10$.

## Claims

1. Substituted aminosulfonyl 6-nitrobenzoic-esters or amides of the formula I

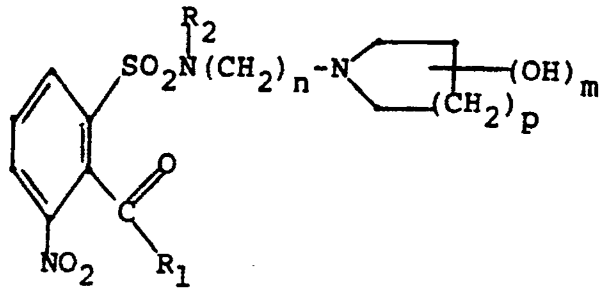

wherein
$R_1$ is hydroxy-alkoxy having 1 - 4 carbon atoms, alkoxy having 1 - 4 carbon atoms, allyloxy or a group having the formula

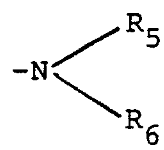

wherein
$R_5$ and $R_6$ are independently from each other hydrogen, alkyl having 1 - 4 carbon atoms or hydroxy-alkyl having 1 - 4 carbon atoms or one of said radicals is allyl and the other one is hydrogen;
$R_2$ is hydrogen, alkyl having 1 - 4 carbon atoms, hydroxy-alkyl having 1 - 4 carbon atoms or allyl;
n is 2 or 3;
m is 0, 1 or 2;
p is 1 or 2
or salts of the compounds of formula I.

2. Compounds of formula I according to claim 1 in which m is 1.

3. Compounds of formula I according to claim 2 characterized in that they are:
the N,N-dimethyl-2-[N-methyl-N-(2-(4-hydroxypiperidino)ethyl)aminosulfonyl]-6-nitrobenzamide,
the methyl 2-[N-(2-(4-hydroxypiperidino)ethyl]aminosulfonyl]-6-nitrobenzoate,
the N,N-dimethyl-2-[N-(2-(4-hydroxypiperidino)ethyl]aminosulfonyl]-6-nitrobenzamide, or
the N,N-dimethyl-2-[N-(2-(3-hydroxypyrrolidino)ethyl)-N-methylaminosulfonyl]-6-nitrobenzamide,
or salts of said compounds.

4. Process for the preparation of compounds of formula I

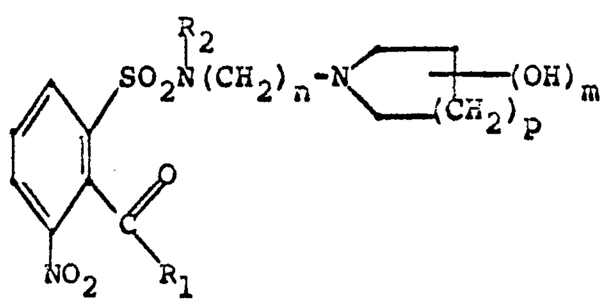

I

according to claim 1 wherein
$R_1$, $R_2$, n, m and p are as defined in claim 1,
or of salts of said compounds of formula I,
characterized in that either
a compound of formula III,

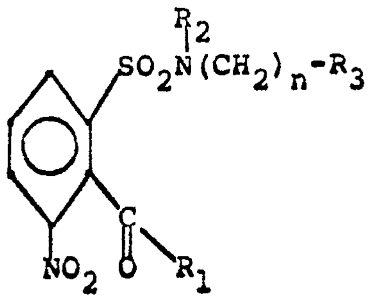

III

wherein $R_3$ is a displaceable group and $R_1$, $R_2$ and n have the same meaning as in claim 1, is reacted in an aprotic solvent with an amine of formula IV

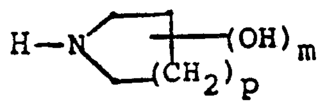

IV

wherein p and m have the same meaning as in claim 1, yielding the compounds of formula I, which are isolated in the free form or as salts thereof,
or that a 2-halosulfonyl compound of formula V

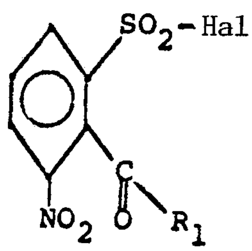

V

wherein
Hal is chlorine and
$R_1$ is as defined in formula I,
is reacted with an amine of formula VI

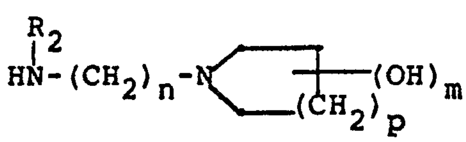

VI

wherein
$R^2$ n, p and m are as defined in claim 1, yielding the compounds of formula I which are isolated in the free form or as salts thereof,
and that optionally a prepared compound of formula I in which
$R_1$ is hydroxy-alkoxy having 1 - 4 carbon atoms or alkoxy having 1 - 4 carbon atoms is further reacted with ammonia or an amine of formula VII

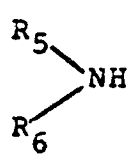

VII

wherein
$R_5$ and $R_6$ are independently from each other hydrogen, alkyl having 1 - 4 carbon atoms or hydroxyalkyl having 1 - 4 carbon atoms or one of said radicals is hydrogen and the other one is, allyl, yielding compounds of formula I in which
$R_1$ is a group having the formula

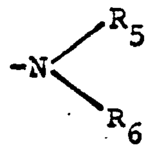

wherein $R_5$ and $R_6$ are as defined in claim 1 and that said benzoic acid amides are isolated in the free form or as salts thereof.

5. Process according to claim 4 wherein in the starting material of formula III the displaceable group $R_3$ is halogen, alkylsulfonyl or arylsulfonyl and wherein the reactions in which either an amine of formula IV or an amine of formula VI takes part are preferably performed in the presence of a base.

6. Process according to claim 4 or 5 characterized in that the compounds according to claims 2 or 3 are prepared.

7. Pharmaceutical composition for enhancing the effect of a therapeutic radiation of hypoxic tumor cells, which composition is characterized in that it contains as pharmaceutically active ingredient a compound of formula I according to claim 1 or a pharmaceutically acceptable salt of the compound of formula I.

8. Pharmaceutical composition according to claim 7 characterized in that it contains as active ingredient a compound according to claim 2 or 3 or a pharmaceutically acceptable salt of said compound.

9. Pharmaceutical composition according to claim 7 or 8 characterized in that it furthermore comprises a pharmaceutically acceptable carrier material.

## Revendications

1. Esters ou amides 6-nitrobenzoïques à substitution aminosulfonyle de formule I

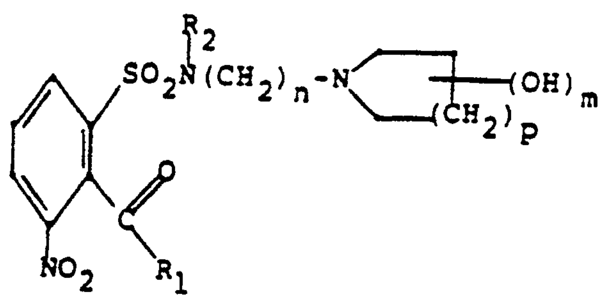

dans laquelle
$R_1$ est un groupe hydroxyalcoxy comportant 1-4 atomes de carbone, alcoxy comportant 1-4 atomes de carbone, allyloxy ou un groupe de formule

$$-N<\begin{matrix}R_5\\R_6\end{matrix}$$

dans laquelle

$R_5$ et $R_6$ sont, indépendamment l'un de l'autre, un atome hydrogène, un groupe alkyle comportant 1-4 atomes de carbone ou hydroxyalkyle comportant 1-4 atomes de carbone, ou l'un desdits radicaux est un groupe allyle et l'autre est un atome d'hydrogène;

$R_2$ est un atome d'hydrogène, un groupe alkyle comportant 1-4 atomes de carbone, hydroxyalkyle comportant 1-4 atomes de carbone ou allyle;

n est égal à 2 ou 3;

m est égal à 0, 1 ou 2;

p est égal à 1 ou 2;

ou sels des composés de formule I.

2. Composés de formule I selon la revendication 1, dans lesquels m est égal à 1.

3. Composés de formule I selon la revendication 2, caractérisés en ce qu'il s'agit : du N,N-diméthyl-2-[N-méthyl-N-(2-(4-hydroxypipéridino)éthyl)aminosulfonyl]-6-nitrobenzamide, du 2-[N-(2-(4-hydroxypipéridino)éthyl)aminosulfonyl]-6-nitrobenzoate de méthyle, du N,N-diméthyl-2-[N-(2-(4-hydroxypipéridino)éthyl)aminosulfonyl]-6-nitrobenzamide ou du N,N-diméthyl-2-[N-(2-(3-hydroxypyrrolidino)éthyl)-N-méthylaminosulfonyl]-6-nitrobenzamide, ou des sels desdits composés.

4. Procédé de préparation de composés de formule I

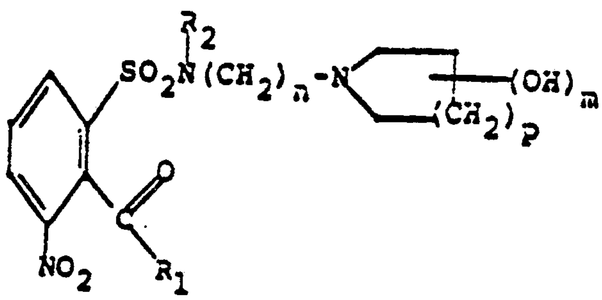

I

selon la revendication 1, dans lesquels $R_1$, $R_2$, n, m et p sont tels que définis dans la revendication 1, ou de sels desdits composés de formule I, caractérisé soit en ce que l'on fait réagir un composé de formule III

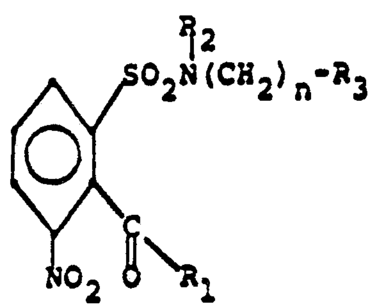

III

dans laquelle $R_3$ est un groupe éliminable et $R_1$, $R_2$ et n sont tels que définis dans la revendication 1, dans un solvant aprotique, avec une amine de formule IV

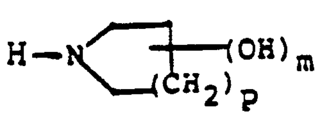

IV

dans laquelle p et m ont la même signification que dans la revendication 1, pour obtenir les composés de formule I, que l'on isole sous forme libre ou sous forme de leurs sels,

soit en ce que l'on fait réagir un composé 2-halogénosulfonyle de formule V

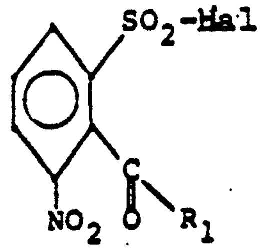

V

dans laquelle
Hal est un atome de chlore et
$R_1$ est tel que défini dans la formule I,
avec une amine de formule VI

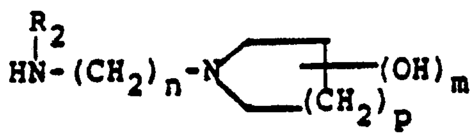

VI

dans laquelle $R_2$, n, p et m sont tels que définis dans la revendication 1, pour obtenir les composés de formule I, que l'on isole sous forme libre ou sous forme de leurs sels, et en ce que l'on fait ensuite éventuellement réagir un composé de formule I préparé, dans lequel
$R_1$ est un groupe hydroxy-alcoxy comportant 1-4 atomes de carbone ou alcoxy comportant 1-4 atomes de carbone, avec de l'ammoniac ou une amine de formule VII

$$\begin{matrix} R_5 \\ R_6 \end{matrix} > NH \qquad VII$$

dans laquelle
$R_5$ et $R_6$ sont, indépendamment l'un de l'autre, un atome hydrogène, un groupe alkyle comportant 1-4 atomes de carbone ou hydroxyalkyle comportant 1-4 atomes de carbone, ou l'un desdits radicaux est un atome d'hydrogène et l'autre est un groupe allyle, pour obtenir les composés de formule I dans lesquels
$R_1$ est un groupe de formule

$$-N < \begin{matrix} R_5 \\ R_6 \end{matrix}$$

dans laquelle $R_5$ et $R_6$ sont tels que définis dans la revendication 1,
et en ce que lesdits amides d'acide benzoïque sont isolés sous forme libre ou sous forme de leurs sels.

5. Procédé selon la revendication 4, dans lequel, dans la substance de départ de formule III, le groupe éliminable $R_3$ est un halogène, un groupe alkylsulfonyle ou arylsulfonyle, et dans lequel les réactions dans lesquelles prend part une amine de formule IV ou une amine de formule VI sont de préférence mises en oeuvre en présence d'une base.

6. Procédé selon la revendication 4 ou 5, caractérisé en ce que l'on prépare les composés selon les revendications 2 ou 3.

7. Composition pharmaceutique permettant d'accroître l'effet d'un rayonnement thérapeutique sur des cellules tumorales hypoxiques, cette composition étant caractérisée en ce qu'elle contient, comme ingrédient pharmaceutiquement actif, un composé de formule I selon la revendication 1 ou un sel pharmaceutiquement acceptable du composé de formule I.

8. Composition pharmaceutique selon la revendication 7, caractérisée en ce qu'elle contient, comme ingrédient actif, un composé selon la revendication 2 ou 3 ou un sel pharmaceutiquement acceptable dudit composé.

9. Composition pharmaceutique selon la revendication 7 ou 8, caractérisée en ce qu'elle comprend en outre un véhicule pharmaceutiquement acceptable.

**Ansprüche**

1. Substituierte Aminosulfonyl-6-nitrobenzoesäureester oder -amide der Formel I

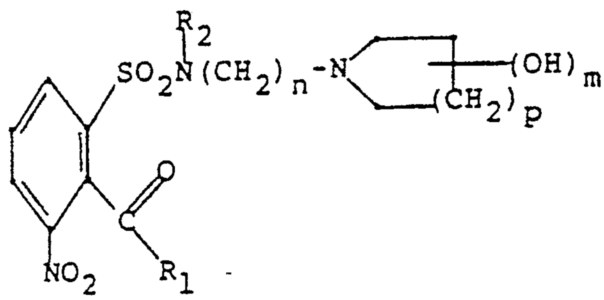

worin
$R_1$ Hydroxyalkoxy mit 1 bis 4 Kohlenstoffatomen, Alkoxy mit 1 bis 4 Kohlenstoffatomen, Allyloxy oder eine Gruppe mit der Formel

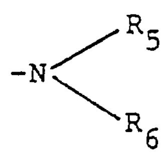

ist, worin
$R_5$ und $R_6$ unabhängig voneinander Wasserstoff, Alkyl mit 1 bis 4 Kohlenstoffatomen oder Hydroxyalkyl mit 1 bis 4 Kohlenstoffatomen sind oder einer dieser Reste Allyl ist und der andere Wasserstoff; $R_2$ Wasserstoff, Alkyl mit 1 bis 4 Kohlenstoffatomen, Hydroxyalkyl mit 1 bis 4 Kohlenstoffatomen oder Allyl ist;
n 2 oder 3 ist;
m 0,1 oder 2 ist;
p 1 oder 2 ist
oder Salze der Verbindungen der Formel I.

2. Verbindung der Formel I nach Anspruch 1, worin m 1 ist.

3. Verbindung der Formel I nach Anspruch 2, dadurch gekennzeichnet, daß sie
das N,N-Dimethyl-2-[N-methyl-N-(2-(4-hydroxypiperdino)ethyl)aminosulfonyl]-6-nitrobenzamid,
das Methyl-2-[N-(2-(4-hydroxypiperidino]ethyl]aminosulfonyl]-6-nitrobenzoat,
das N,N-Dimethyl-2-[N-(2-(4-hydroxypiperidino)ethyl]-aminosulfonyl]-6-nitrobenzamid oder
das N,N-Dimethyl-2-[N-(2-(3-hydroxypyrrolidino)ethyl)-N-methylaminosulfonyl]-6-nitrobenzamid

oder Salze dieser Verbindungen sind.

4. Verfahren zur Herstellung von Verbindungen der Formel I

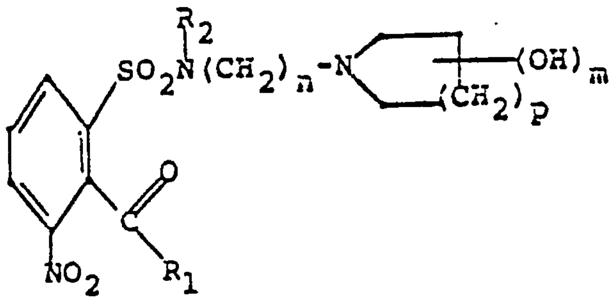

I

nach Anspruch 1, worin
$R_1$, $R_2$, n, m und p wie in Anspruch 1 definiert sind, oder von Salzen dieser Verbindungen der Formel I, dadurch gekennzeichnet, daß entweder eine Verbindung der Formel III

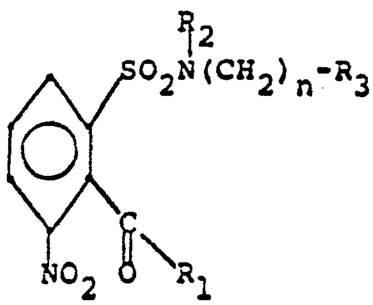

III

worin $R_3$ eine austauschbare Gruppe ist und $R_1$, $R_2$ und n die gleiche Bedeutung haben wie in Anspruch 1, in einem aprotischen Lösungsmittel mit einem Amin der Formel IV

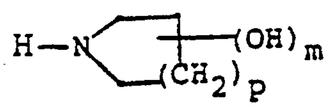

IV

worin p und m die gleiche Bedeutung haben wie in Anspruch 1, unter Erhalt der Verbindungen der Formel I, die in der freien Form oder in Form Salze isoliert werden,
oder daß eine 2-Halogensulfonylverbindung der Formel V

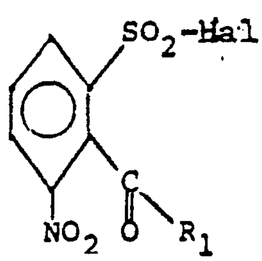

V

worin
Hal Chlor ist und
$R_1$ wie in Formel I definiert ist,
mit einem Amin der Formel VI

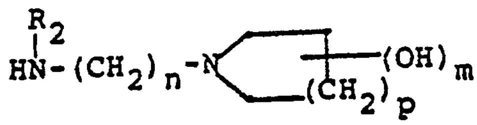

VI

worin
$R_2$, n, p und m wie in Anspruch 1 definiert sind, unter Erhalt der Verbindung der Formel I, die in der freien Form oder in Form ihrer Salze isoliert werden, umgesetzt wird, und daß ggf. eine hergestellte Verbindung der Formel I, worin
$R_1$ Hydroxyalkoxy mit 1 bis 4 Kohlenstoffamtomen oder Alkoxy mit 1 bis 4 Kohlenstoffatomen ist, weiter mit Ammoniak oder einem Amin der Formel VII

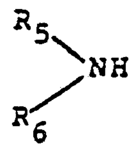

VII

worin
$R_5$ und $R_6$ unabhängig voneinander Wasserstoff, Alkyl mit 1 bis 4 Kohlenstoffatomen oder Hydroxyalkyl mit 1 bis 4 Kohlenstoffatomen sind oder einer dieser Reste Wasserstoff ist und der andere Allyl ist, unter Erhalt von Verbindungen der Formel I, worin $R_1$ eine Gruppe der Formel ist

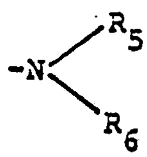

worin $R_5$ und $R_6$ wie in Anspruch 1 definiert sind, umgesetzt wird,
und daß die Benzoesäureamide in der freien Form oder in Form ihrer Salze isoliert werden.

5. Verfahren nach Anspruch 4, worin im Ausgangsmaterial der Formel III die austauschbare Gruppe $R_3$ Halogen, Alkylsulfonyl, Arylsulfonyl ist und worin die Reaktionen, in denen entweder ein Amin der Formel IV oder ein Amin der Formel VI teilnimmt, vorzugsweise in Gegenwart einer Base durchgeführt werden.

6. Verfahren nach Anspruch 4 oder 5, dadurch gekennzeichnet, daß Verbindungen nach den Ansprüchen 2 oder 3 hergestellt werden.

7. Pharmazeutische Zusammensetzung zur Verstärkung der Wirksamkeit einer therapeutischen Bestrahlung von hypoxischen Tumorzellen, welche Zusammensetzung dadurch gekennzeichnet ist, daß sie als pharmazeutisch wirksamen Bestandteil eine Verbindung der Formel I nach Anspruch 1 oder ein pharmazeutisch annehmbares Salz der Verbindung der Formel I enthält.

8. Pharmazeutische Zusammensetzung nach Anspruch 7, dadurch gekennzeichnet, daß sie wirksamen Bestandteil eine Verbindung nach Anspruch 2 oder 3 oder ein pharmazeutisch annehmbares Salz dieser Verbindung enthält.

9. Pharmazeutische Zusammensetzung nach Anspruch 7 oder 8, dadurch gekennzeichnet, daß sie weiterhin ein pharmazeutisch annehmbares Trägermaterial enthält.